(19) Europäisches Patentamt
European Patent Office
Office européen des brevets

(11) EP 2 243 530 A1

(12) **EUROPÄISCHE PATENTANMELDUNG**

(43) Veröffentlichungstag:
**27.10.2010 Patentblatt 2010/43**

(51) Int Cl.:
***B01D 3/40*** *(2006.01)*

(21) Anmeldenummer: **09158388.0**

(22) Anmeldetag: **21.04.2009**

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**
Benannte Erstreckungsstaaten:
**AL BA RS**

(71) Anmelder: **LANXESS Deutschland GmbH**
**51369 Leverkusen (DE)**

(72) Erfinder:
- **Kuhlmann, Sven**
  **50733 Köln (DE)**
- **Weber, Hans-Martin**
  **51373 Leverkusen (DE)**
- **Dummersdorf, Hans-Ulrich**
  **51399 Burscheid (DE)**
- **Hallenberger, Kaspar**
  **51375 Leverkusen (DE)**

(54) **Verfahren zur Trennung aromatischer Verbindungen**

(57) Die Erfindung betrifft ein Verfahren zur destillativen Trennung von Stoffgemischen unter Einsatz von ionischen Flüssigkeiten als Extraktionsmittel.

EP 2 243 530 A1

## Beschreibung

Die Erfindung betrifft ein Verfahren zur destillativen Trennung von Stoffgemischen unter Einsatz von ionischen Flüssigkeiten als Extraktionsmittel.

Die Rektifikation ist eine in der Technik verbreitet angewandte Methode um Stoffgemische voneinander zu trennen. Der erforderliche Aufwand für die Trennung eines Stoffgemisches bei der Rektifikation wird durch den sogenannten Trennfaktor $\alpha$, dem Verhältnis der Verteilungskoeffizienten der beteiligten Komponenten in der dampfförmigen Phase und in der Flüssigphase wiedergegeben. Je höher der Abstand des Trennfaktors vom Wert eins ist, desto wirtschaftlicher lässt sich in der Regel das Stoffgemisch trennen.

Viele Stoffgemische, deren Komponenten nur geringe Siedepunktsunterschiede aufweisen oder gar Azeotrope bilden, sind durch konventionelle Rektifikation nur schwer bzw. gar nicht trennbar. Dies trifft beispielsweise auf die Trennung von Stellungsisomeren mehrfach substituierter Verbindungen, wie insbesondere von Stellungsisomeren mehrfach substituierter aromatischer Verbindungen zu. In solchen Fällen wird dem zu trennenden Stoffgemisch typischerweise ein Zusatzstoff, ein sogenannter Entrainer zugesetzt, der über selektive Wechselwirkungen mit den Komponenten des Stoffgemisches das Dampf-Flüssig-Phasengleichgewicht in der Art beeinflusst, dass höhere Trennfaktoren erreicht werden, als ohne Zusatz der Entrainer. Typischerweise sinkt dadurch der Energie- bzw. Kostenaufwand für die Trennung des eingesetzten Stoffgemisches bzw. eine Trennung wird überhaupt erst möglich. In jüngster Zeit sind auch ionische Flüssigkeiten als Entrainer Gegenstand von Untersuchungen geworden (siehe auch DE 10136614 A, DE 10154052 A und Chem. Ing. Tech. 2003, 75, 1148-1149).

Aus der Literatur ist eine Vielzahl von Entrainern für die Trennung von aromatischen Verbindungen bekannt.

So beschreibt DE 10251191 A die Verwendung von Alkyl- und Alkylenphosphaten sowie Phosphinoxiden zur Trennung von Gemischen, enthaltend m- und p-Dichlorbenzol, wobei Trennfaktoren von bis zu 1,23 erreicht werden. Weiterhin werden andere, allerdings weniger geeignete Entrainer offenbart.

EP 1 372 807 A beschreibt die Verwendung von ionischen Flüssigkeiten wie beispielsweise Imidazolium-tetrafluoroboraten, -hexafluorophosphaten, trifluormethylsulfonylimiden und Methansulfonaten für die Trennung von aliphatischen Verbindungen.

Vor dem Hintergrund des vorstehend genannten Standes der Technik bestand die Aufgabe, ein Verfahren bereitzustellen, mit dem aromatische Verbindungen mit guten Trennfaktoren effizient getrennt werden können.

Gegenstand der Erfindung ist nun ein Verfahren zur Trennung von Mischungen aromatischer Verbindungen mittels extraktiver Rektifikation, das dadurch gekennzeichnet ist, dass die Rektifikation in Gegenwart von ionischen Flüssigkeiten, bevorzugt in Gegenwart von ionischen Flüssigkeiten, deren Kation Stickstoff oder Phosphor enthält; besonders bevorzugt in Gegenwart von ionischen Flüssigkeiten, deren Kation einen Stickstoff enthaltenden Heterocyclus umfasst und ganz besonders bevorzugt in Gegenwart einer oder mehrere Verbindungen der Formel (I) durchgeführt wird,

$$Kat^+ \quad An^- \qquad (I)$$

in der

- $Kat^+$ für einen substituierten, Stickstoff enthaltenden Heterocyclus, bevorzugt für ein Pyridinium- oder Imidazolium-Kation steht und
- $An^-$ für Phosphat oder ein organisches Phosphat steht.

Der Begriff ionische Flüssigkeit bedeutet im Rahmen der Erfindung eine Verbindung, die mindestens eine positive und eine negative Ladung aufweist, insgesamt jedoch ladungsneutral ist, und unter den Rektifikationsbedingungen flüssig ist, bevorzugt einen Schmelzpunkt unter 200°C aufweist, besonders bevorzugt einen Schmelzpunkt von unter 100°C.

Bevorzugte Verbindungen der Formel (I) sind solche der Formel (Ia)

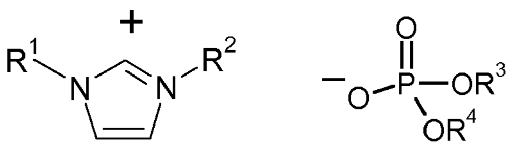

(Ia)

in der
$R^1$ und $R^2$ sowie $R^3$ und $R^4$ jeweils unabhängig voneinander für Wasserstoff, $C_1$-$C_{12}$-Alkyl-, $C_5$-$C_{14}$-Aryl, $C_6$-$C_{20}$-Arylalkyl-, $C_2$-$C_{12}$-Alkenyl oder $C_2$-$C_{12}$-Alkylen stehen oder $R^1$ und $R^2$ bzw. $R^3$ und $R^4$ zusammen für $C_2$-$C_{12}$-Alkylen oder $C_2$-$C_{12}$-Alkenylen stehen.

$C_1$-$C_{12}$-Alkyl beziehungsweise $C_2$-$C_{12}$-Alkylen beziehungsweise $C_2$-$C_{12}$-Alkenylen bedeutet jeweils unabhängig einen geradkettigen, ganz oder teilweise cyclischen, verzweigten oder unverzweigten $C_1$-$C_{12}$-Alkyl- beziehungsweise $C_2$-$C_{12}$-Alkylen- beziehungsweise $C_2$-$C_{12}$-Alkenylen-Rest, wobei die genannten Reste gegebenenfalls weiterhin durch $C_1$-$C_4$-Alkoxyreste substituiert sein können.

Gleiches gilt für den nichtaromatischen Teil eines $C_6$-$C_{20}$-Arylalkyl-Restes.

$C_1$-$C_{12}$-Alkyl steht beispielsweise für Methyl, Ethyl, n-Propyl, iso-Propyl, n-Butyl, sec.-Butyl und tert.-Butyl, n-Pentyl, 1-Methylbutyl, 2-Methylbutyl, 3-Methylbutyl, neo-Pentyl, 1-Ethylpropyl, cyclo-Hexyl, cyclo-Pentyl, n-Hexyl, 1,1-Dimethylpropyl, 1,2-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, 1-Ethyl-2-methylpropyl, n-Heptyl, n-Octyl, 2-Ethylhexyl, Adamantyl, die isomeren Menthyle, n-Nonyl, n-Decyl oder n-Dodecyl.

$C_1$-$C_4$-Alkoxy steht beispielsweise für Methoxy, Ethoxy, 2-Methoxyethoxy, n-Propoxy, iso-Propoxy, n-Butoxy, sec.-Butoxy oder tert.-Butoxy.

$C_2$-$C_{12}$-Alkenyl steht beispielsweise für Vinyl, 1-Propenyl, iso-Propenyl, 1-Butenyl, 2-Butenyl, 1-Pentenyl, 2-Pentenyl, 2-Methyl-1-butenyl, 2-Methyl-2-butenyl, 3-Methyl-1-buteny, 1-Hexenyl, 1-Heptenyl, 1-Octenyl oder 2-Octenyl.

$C_5$-$C_{14}$-Aryl bedeutet jeweils unabhängig einen heteroaromatischen Rest mit 5 bis 14 Gerüstkohlenstoffatomen, in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können, vorzugsweise jedoch für einen carbocyclischen aromatischen Rest mit 6 bis 14 Gerüstkohlenstoffatomen.

Bevorzugte Beispiele für carbocyclische aromatische Reste mit 6 bis 14 Gerüstkohlenstoffatomen sind zum Beispiel Phenyl, Naphtyl, Phenanthrenyl, Anthracenyl oder Fluorenyl, heteroaromatische Reste mit 5 bis 14 Gerüstkohlenstoffatomen in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können sind beispielsweise Pyridinyl, Oxazolyl, Benzofuranyl, Dibenzofuran-yl oder Chinolinyl.

Weiterhin kann der carbocyclische aromatische Rest oder heteroaromatische Rest mit bis zu fünf gleichen oder verschiedenen Substituenten pro Cyclus substituiert sein, die ausgewählt sind aus der Gruppe Chlor, Fluor, $C_1$-$C_{12}$-Alkyl, $C_1$-$C_{12}$-Halogenalkyl und $C_1$-$C_{12}$-Alkoxy.

Besonders bevorzugt steht $C_5$-$C_{14}$-Aryl für Phenyl.

Gleiches gilt für den aromatischen Teil eines $C_6$-$C_{20}$-Arylalkyl-Restes. Bevorzugt steht $C_6$-$C_{20}$-Arylalkyl für Benzyl.

Weiterer Gegenstand der Erfindung ist die Verwendung von ionischen Flüssigkeiten, wie insbesondere solchen Verbindungen der Formeln (I) und (Ia) zur Trennung von Mischungen enthaltend im Wesentlichen aromatische Verbindungen.

Besonders bevorzugt stehen in der Formel (Ia) die Reste $R^1$, $R^2$, $R^3$ und $R^4$ jeweils unabhängig voneinander oder jeweils identisch für einen Rest aus der Reihe: Methyl, Ethyl, n-Propyl, n-Butyl, n-Hexyl, 2-Ethylhexyl oder n-Octyl.

Besonders bevorzugte Verbindungen der Formeln (I) und (Ia) sind 1,3-Dimethylimidazolium-dimethylphosphat, 1-Ethyl-3-methylimidazolium-dimethylphosphat, 1,3-Diethyl-imidazoliumdiethylphosphat, 1-Ethyl-3-methylimidazolium-diethylphosphat.

Die Verbindungen der Formeln (I) bzw. (Ia) und ihre Herstellung sind grundsätzlich bekannt und beispielsweise in Ionic Liquids in Synthesis, ISBN 3-527-31239-0 und 978-3-527-31239-9 offenbart.

Der Begriff aromatische Verbindungen umfasst im Sinne der Erfindung alle Verbindungen enthaltend aromatische Reste, die zu mindestens 99 % thermisch unzersetzt bei irgendeiner Temperatur von 250°C oder weniger und irgendeinem Druck von mehr als 0,1 hPa, bevorzugt mehr als 0,5 hPa destilliert werden können.

Als Mischungen enthaltend im Wesentlichen aromatische Verbindungen eignen sich insbesondere Mischungen, die im wesentlichen Stellungsisomere mehrfach substituierter aromatischer Verbindungen enthalten, wie beispielsweise Mischungen, die im Wesentlichen enthalten: o-, m- und p-Dichlorbenzol; o-, m- und p-Kresol; o-, m- und p-Xylol; o-, m- und p-Chlortoluol; o-, m- und p-Nitrotoluol; o-, m- und p-Nitrochlorbenzol; o-, m- und p-Chloranilin; o-, m- und p-Toluidin; o-, m- und p-Trifluormethoxynitrobenzol, 1,2,3-, 1,2,4 und 1,3,5-Trichlorbenzol; 2,3-, 2,4-, 2,5-, 2,6-, 3,4- und 3,5-Dichlortoluol; 2,3-, 2,4-, 2,5-, 2,6-, 3,4- und 3,5-Dichlornitrobenzol; 2,3-, 2,4-, 2,5-, 2,6-, 3,4- und 3,5-Dichloranilin; 2,3-, 2,4-, 2,5-, 2,6-, 3,4- und 3,5-Dinitrotoluol oder Styrol und Ethylbenzol.

Bevorzugt sind Mischungen aromatischer Verbindungen, die im Wesentlichen Substituenten in m- und p-Stellung enthalten. Besonders bevorzugt sind Mischungen, die im Wesentlichen mund p-Dichlorbenzol enthalten.

Das jeweilige Verhältnis der zu trennenden Komponenten im Ausgangsgemisch ist dabei prinzipiell nicht begrenzt. Der Begriff "im Wesentlichen" soll im Sinne der Erfindungen bedeuten, dass der Anteil sonstiger Komponenten über die zu trennenden aromatischen Komponenten oder über die zwei zu trennenden aromatischen Komponenten hinaus 0 bis 20, bevorzugt 0 bis 10 Gew.-% und besonders bevorzugt 0 bis 5 Gew.-% betragen kann.

Besonders bevorzugt eignet sich das erfindungsgemäße Verfahren zur Trennung von m- und p-Dichlorbenzol.

Im Allgemeinen wird das erfindungsgemäße Verfahren so durchgeführt, dass die in die Extraktivdestillation eingespeiste, zu trennende Mischung zunächst auf eine Temperatur gebracht wird oder diese aufweist, die in Abhängigkeit vom gewählten Kolonnendruck zwischen 20 und 150°C und vorzugsweise zwischen 80 und 120°C liegt. In einer bevorzugten Ausführungsform liegt die Sumpftemperatur in der Rektifikationskolonne bei 180°C oder weniger, besonders bevorzugt bei 130°C oder weniger.

In einer bevorzugten Ausführungsform wird die Trennung in einer Rektifikationskolonne durchgeführt, wobei der Druck am Kolonnenkopf im allgemeinen und abhängig vom zu trennenden Gemisch beispielsweise im Bereich von 5 bis 500 hPa liegt und bevorzugt 5 bis 200 hPa betragen kann.

Die Druckdifferenz zwischen Kolonnensumpf und Kolonnenkopf kann beispielsweise 0 bis 200, bevorzugt 0 bis 100 hPa betragen.

Die Anzahl der theoretischer Trennstufen kann beispielsweise 5 bis 1000, vorzugsweise 20 bis 200 betragen.

Das Gewichtsverhältnis von Rücklauf zu Destillatentnahme kann beispielsweise 0,5:1 bis 20:1, besonders bevorzugt 2:1 bis 8:1 betragen.

Das Gewichtsverhältnis von ionischen Flüssigkeiten bzw. der Verbindungen der Formeln (I) bzw. (Ia) zur Menge des zu trennenden Stoffgemisches, das der Rektifikationskolonne zugeführt wird, kann beispielsweise 2:1 bis 40:1, bevorzugt 2:1 bis 8:1 betragen.

Zur Rückgewinnung der ionischen Flüssigkeiten bzw. der Verbindungen der Formeln (I) und (Ia) zum späteren erneuten Einsatz in der Extraktivrektifikation erfolgt zum Beispiel durch destillative Abtrennung vom restlichen Produkt. Vorzugsweise erfolgt die destillative Abtrennung in zwei Trennstufen ohne Rücklauf. Dabei wird in der ersten Trennstufe bei einem Druck im Bereich von 5 bis 200 hPa und bevorzugt 5 bis 50 hPa das restliche Produkt weitestgehend abgetrennt, wobei unter dem Begriff "weitestgehend" hierbei eine mehr als 90%-ige Abtrennung, vorzugsweise eine mehr als 95%-ige Abtrennung zu verstehen ist. In einer zweiten Trennstufe erfolgt danach eine weitere Abtrennung bei einem niedrigeren Druck als in der ersten Trennstufe von beispielsweise 1 bis 20 hPa und bevorzugt 1 bis 5 hPa. Die Kondensation der Produktbrüden findet zweckmäßigerweise nach einer Druckanhebung auf beispielsweise 5 bis 200 hPa und bevorzugt 5 bis 50 hPa statt.

Der besondere Vorteil des erfindungsgemäßen Verfahrens ist, dass in effizienter Weise Mischungen aromatischer Verbindungen derart voneinander getrennt werden können, dass die einzelnen Komponenten mit hohen Reinheiten erhalten werden können.

Dabei kann der spezifische Energieverbrauch deutlich gesenkt werden und die Abmaße der verwendeten Trennapparate, d.h. das notwendige Apparatevolumen je erforderliche Trennstufe, reduzieren sich erheblich.

## Beispiel

Trennung von Dichlorbenzol-Gemischen

Daten zur Wirksamkeit der erfindungsgemäßen Extraktionsmittel sind in nachfolgender Tabelle zusammengestellt. Diese Trennfaktoren wurden bei 120°C nach der Headspacemethode gemessen (siehe auch Verfahrenstechnik 1974, 8, 12, 343-347)

Die Messungen wurden bei einer Konzentration in der Flüssigkeit von 10 Mol-% m-Dichlorbenzol, 10 Mol-% p-Dichlorbenzol und 80 Mol-% Extraktionsmittel ausgeführt. Zum Vergleich der Wirksamkeit dieser Extraktionsmittel sind auch die entsprechenden Messwerte der bekannten Extraktionsmittel Triethylphosphat und Sulfolan in die Tabelle aufgenommen worden.

| Extraktionsmittel | Trennfaktor |
|---|---|
| 1,3-Diethylimidazolium-diethylphosphat | 1,34 |
| 1,3-Ethylmethylimidazolium-dimethylphosphat | 1,31 |
| 1,3-Dimethylimidazolium-dimethylphosphat | 1,29 |
| 1,3-Ethlymethylimidazolium-diethylphosphat | 1,27 |
| | |
| Stand der Technik: | |
| Triethylphosphat | 1,23 |
| Sulfolan | 1,14 |

Die Prüfungen zeigen, dass die erfindungsgemäßen Extraktionsmittel im Vergleich zum Stand der Technik deutlich überlegen sind.

Weitere Untersuchungen haben gezeigt, dass die erfindungsgemäßen Extraktionsmittel eine hohe thermische Stabilität zeigen auch in Gegenwart von die thermische Zersetzung stark katalysierenden Substanzen, wie Rost und anderen Schwermetallverbindungen, was einerseits für einen sicheren Betrieb der Trennanlagen von erheblicher Bedeutung ist und andererseits eine lange und verlustfreie Verwendung des Trennmittels garantiert.

Destillationsbeispiel

Eine Extraktionskolonne mit 100 theoretischen Trennstufen wurde mit dem zu trennenden Gemisch bestehend aus 58 Gew.-% m-Dichlorbenzol und 38 Gew.-% p-Dichlorbenzol mit einer Temperatur von 100°C und dem Extraktionsmittel 1,3-Diethylimidazolium-diethylphosphat mit einer Temperatur von 60°C gespeist. Dabei wurde der Entrainer oberhalb des Dichlorbenzol-Gemisches eingespeist. Das Verhältnis von Dichlorbenzol zu Entrainer lag bei 1:5.

Der Druck am Kolonnenkopf betrugt 100 hPa und im Kolonnensumpf 140 hPa. Das Extraktionsmittel verliess den Kolonnensumpf mit einer Temperatur von 158°C. Im Sumpfstrom waren mehr als 99 Gew.-% des eingesetzten p-Dichlorbenzol und weniger als 50 Gew.-% des eingesetzten m-Dichlorbenzol enthalten. Im Kopfstrom der Kolonne wurde hochreines Zielprodukt mit einem Gehalt von mehr als 99 Gew.-% m-Dichlorbenzol entnommen, wobei das Rücklaufverhältnis (Rückflussmenge/Destillat) bei 6:1 lag.

## Patentansprüche

1. Verfahren zur Trennung von Mischungen aromatischer Verbindungen mittels extraktiver Rektifikation, **dadurch gekennzeichnet, dass** die Rektifikation in Gegenwart von ionischen Flüssigkeiten durchgeführt wird.

2. Verfahren nach Anspruch 1, **dadurch gekennzeichnet dass** als ionische Flüssigkeiten solche eingesetzt werden, deren Kation Stickstoff oder Phosphor enthält.

3. Verfahren nach Anspruch 1 oder 2, **dadurch gekennzeichnet dass** als ionische Flüssigkeiten solche eingesetzt werden, deren Kation einen Stickstoff enthaltenden Heterocyclus umfasst.

4. Verfahren nach einem der Ansprüche 1 bis 3, **dadurch gekennzeichnet dass** als ionische Flüssigkeiten solche der Formel (I) eingesetzt werden,

$$Kat^{+} \quad An^{-} \qquad (I)$$

   in der

   $Kat^+$ für einen substituierten, Stickstoff enthaltenden Heterocyclus steht und
   $An^-$ für Phosphat oder ein organisches Phosphat steht.

5. Verfahren nach einem der Ansprüche 1 bis 4, **dadurch gekennzeichnet dass** als ionische Flüssigkeiten solche der Formel (Ia) eingesetzt werden,

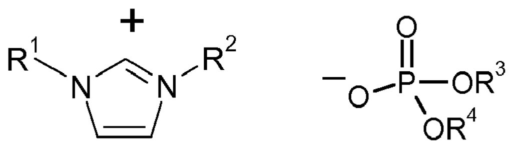

(Ia)

   in der
   $R^1$ und $R^2$ sowie $R^3$ und $R^4$ jeweils unabhängig voneinander für Wasserstoff, $C_1$-$C_{12}$-Alkyl-, $C_5$-$C_{14}$-Aryl, $C_6$-$C_{20}$-Arylalkyl-, $C_2$-$C_{12}$-Alkenyl oder $C_2$-$C_{12}$-Aklen stehen oder $R^1$ und $R^2$ bzw. $R^3$ und $R^4$ zusammen für $C_2$-$C_{12}$-Alkylen oder $C_2$-$C_{12}$-Alkenylen stehen.

**6.** Verfahren nach Anspruch 5, **dadurch gekennzeichnet, dass** in der Formel (Ia) die Reste $R^1$, $R^2$, $R^3$ und $R^4$ jeweils unabhängig voneinander oder jeweils identisch für einen Rest aus der Reihe: Wasserstoff, Methyl, Ethyl, n-Propyl, n-Butyl, n-Hexyl, 2-Ethylhexyl oder n-Octyl stehen.

**7.** Verfahren nach Anspruch 6" **dadurch gekennzeichnet, dass** die Verbindungen der Formel (Ia) ausgewählt sind aus der Gruppe: 1,3-Dimethylimidazolium-dimethylphosphat, 1-Ethyl-3-methylimidazolium-dimethylphosphat, 1,3-Diethylimidazolium-diethylphosphat und 1-Ethyl-3-methylimidazolium-diethylphosphat.

**8.** Verfahren nach einem der Ansprüche 1 bis 7, **dadurch gekennzeichnet, dass** als Mischungen aromatischer Verbindungen Mischungen verwendet werden, die im wesentlichen Stellungsisomere mehrfach substituierter aromatischer Verbindungen enthalten.

**9.** Verfahren nach einem der Ansprüche 1 bis 8, **dadurch gekennzeichnet, dass** die Sumpftemperatur in der extraktiven Rektifikation 180°C oder weniger beträgt.

**10.** Verfahren nach einem der Ansprüche 1 bis 9, **dadurch gekennzeichnet, dass** die Anzahl der theoretischer Trennstufen bei der extraktiven Rektifikation 5 bis 1000 beträgt.

**11.** Verfahren nach einem der Ansprüche 1 bis 10, **dadurch gekennzeichnet, dass** das Gewichtsverhältnis von Rücklauf zu Destillatentnahme bei der extraktiven Rektifikation 0,5:1 bis 20:1 beträgt.

**12.** Verwendung von ionischen Flüssigkeiten zur destillativen Trennung von Mischungen enthaltend im Wesentlichen aromatische Verbindungen.

Europäisches Patentamt
European Patent Office
Office européen des brevets

# EUROPÄISCHER RECHERCHENBERICHT

Nummer der Anmeldung
EP 09 15 8388

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X<br><br><br><br><br>Y | WO 2005/019137 A (BASF AG [DE]; MAASE MATTHIAS [DE]; BUDICH MANUEL [DE]; GROSSMANN GEORG) 3. März 2005 (2005-03-03)<br>* Seite 4, Zeile 11 - Zeile 14; Ansprüche 1,8,9 * | 1-4,9-12<br><br><br><br><br>5-7 | INV.<br>B01D3/40 |
| X | WO 02/30878 A (UNIV BELFAST [GB]; EARLE MARTYN JOHN [GB]; KATDARE SUHAS PRABHAKAR [GB) 18. April 2002 (2002-04-18)<br>* Ansprüche 1,10,11,17; Tabelle 1 * | 1,8,12 | |
| Y | KATO R ET AL: "Activity coefficients at infinite dilution of various solutes in the ionic liquids [MMIM]<+>[CH3SO4]<->, [MMIM]<+>[CH3OC2H4SO4]<->, [MMIM]<+>[(CH3)2PO4]<->, [C5H5NC2H5]<+>[(CF3SO2)2N]<-> and [C5H5NH]<+>[C2H5OC2H4OSO3]<->"<br>FLUID PHASE EQUILIBRIA, ELSEVIER,<br>Bd. 226, 10. Dezember 2004 (2004-12-10), Seiten 37-44, XP004917574<br>ISSN: 0378-3812<br>Seite 44 Konklusion* Tabelle 1 * | 5-7 | **RECHERCHIERTE SACHGEBIETE (IPC)**<br>B01D<br>C07C |
| A | EP 1 854 786 A (BP P L C [GB])<br>14. November 2007 (2007-11-14)<br>* Absatz [0016]; Ansprüche 1,9,14 * | 1-12 | |
| A | US 2003/125599 A1 (BOUDREAU LAURA C [US] ET AL) 3. Juli 2003 (2003-07-03)<br>* Zusammenfassung; Anspruch 30 * | 1-12 | |
| A | DE 10 2007 041416 A1 (BASF SE [DE]; UNIV SIEGEN [DE]) 5. März 2009 (2009-03-05)<br>* Zusammenfassung *<br>* Absätze [0016], [0024], [0068]; Ansprüche 1,4 * | 1-12 | |

-/--

3

Der vorliegende Recherchenbericht wurde für alle Patentansprüche erstellt

| Recherchenort | Abschlußdatum der Recherche | Prüfer |
|---|---|---|
| Den Haag | 4. September 2009 | Weber, Christian |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

Europäisches Patentamt
European Patent Office
Office européen des brevets

# EUROPÄISCHER RECHERCHENBERICHT

Nummer der Anmeldung
EP 09 15 8388

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| D,A | WO 02/074718 A (BASF AG [DE]; ARLT WOLFGANG [DE]; SEILER MATTHIAS [DE]; JORK CARSTEN [) 26. September 2002 (2002-09-26) * Zusammenfassung * ----- | 1-12 | |
| D,A | DE 101 54 052 A1 (CARL V OSSIETZKY UNI OLDENBURG [DE]) 10. Juli 2003 (2003-07-10) * Zusammenfassung; Ansprüche 1,4; Abbildungen 1,3,5,6 * ----- | 1-12 | |
| | | | RECHERCHIERTE SACHGEBIETE (IPC) |

Der vorliegende Recherchenbericht wurde für alle Patentansprüche erstellt

| Recherchenort | Abschlußdatum der Recherche | Prüfer |
|---|---|---|
| Den Haag | 4. September 2009 | Weber, Christian |

3

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 09 15 8388

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

04-09-2009

| Im Recherchenbericht angeführtes Patentdokument | Datum der Veröffentlichung | Mitglied(er) der Patentfamilie | Datum der Veröffentlichung |
|---|---|---|---|
| WO 2005019137 A | 03-03-2005 | AT 395316 T | 15-05-2008 |
| | | CN 1826302 A | 30-08-2006 |
| | | DK 1648845 T3 | 01-09-2008 |
| | | EP 1648845 A1 | 26-04-2006 |
| | | ES 2305787 T3 | 01-11-2008 |
| | | JP 2006528599 T | 21-12-2006 |
| | | KR 20060076269 A | 04-07-2006 |
| | | US 2007193952 A1 | 23-08-2007 |
| WO 0230878 A | 18-04-2002 | AT 284384 T | 15-12-2004 |
| | | AU 9396901 A | 22-04-2002 |
| | | CA 2425168 A1 | 18-04-2002 |
| | | DE 60107706 D1 | 13-01-2005 |
| | | DE 60107706 T2 | 15-12-2005 |
| | | EP 1324982 A1 | 09-07-2003 |
| | | JP 2004511461 T | 15-04-2004 |
| | | US 2004242932 A1 | 02-12-2004 |
| EP 1854786 A | 14-11-2007 | KEINE | |
| US 2003125599 A1 | 03-07-2003 | AU 2002360834 A1 | 30-07-2003 |
| | | WO 03059483 A2 | 24-07-2003 |
| DE 102007041416 A1 | 05-03-2009 | WO 2009027250 A2 | 05-03-2009 |
| WO 02074718 A | 26-09-2002 | AT 279248 T | 15-10-2004 |
| | | AU 2002304855 A1 | 03-10-2002 |
| | | BR 0208176 A | 23-03-2004 |
| | | CA 2440528 A1 | 26-09-2002 |
| | | CN 1524006 A | 25-08-2004 |
| | | EP 1372807 A2 | 02-01-2004 |
| | | ES 2231699 T3 | 16-05-2005 |
| | | JP 2004525924 T | 26-08-2004 |
| | | MX PA03007633 A | 04-12-2003 |
| | | TW 248828 B | 11-02-2006 |
| | | US 2004133058 A1 | 08-07-2004 |
| DE 10154052 A1 | 10-07-2003 | KEINE | |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 10136614 A
- DE 10154052 A
- DE 10251191 A
- EP 1372807 A


### In der Beschreibung aufgeführte Nicht-Patentliteratur


- *Chem. Ing. Tech.,* 2003, vol. 75, 1148-1149
- *Verfahrenstechnik,* 1974, vol. 8 (12), 343-347